# EUROPEAN PATENT APPLICATION

(11) **EP 2 893 941 A1**
(43) Date of publication of application: **15.07.2015**
(21) Application number: 13834973.3
(22) Date of filing: 27.02.2013
(51) Int. Cl.: A61L 27/00, A61L 15/00, A61L 17/00, A61L 31/00, A61L 33/00, C08G 63/183, D01F 6/62

(54) **MEDICAL MATERIAL FOR LONG-TERM IN VIVO IMPLANTATION USE WHICH IS MADE FROM ULTRAFINE FIBER**

(30) Priority: 07.09.2012 JP 2012197055
(71) Applicant: NICEM LTD, Yokohama 236-0005 (JP)
(72) Inventor: NOISHIKI, Yasuharu, Yokohama-shi Kanagawa 236-0005 (JP)
(74) Representative: Raynor, Stuart Andrew
(86) International application number: PCT/JP2013/056043
(87) International publication number: WO 2014/038219

(57) **Abstract**

A medical material for in vivo implantation use, characterized in that a polyethylene terephthalate ultrafine fiber is provided in at least a part of the medical material, wherein the polyethylene terephthalate ultrafine fiber is produced by spinning a polyethylene terephthalate chip, which is produced using germanium or titanium oxide as a catalyst, by a direct spinning method and then stretching the spun product and has a single fiber fineness of 0.8dtex or less.

## Description

### FIELD OF THE INVENTION

The present invention relates to a medical material made of polyethylene terephthalate ultrafine fibers which do not cause foreign body reaction or calcification, has good pliability and easy handling when implanted in vivo for long period of time.

### DESCRIPTION OF PRIOR ARTS

Since safety of medical implants made of polyethylene terephthalate (hereinafter, can be shortened to polyester) fibers in vivo was shown in 1957 (Patent document 1), polyester fibers have been widely used in clinic as a raw material for artificial vascular grafts, patch materials or suture threads. It was also proved that polyester itself does not cause foreign body reaction (Patent document 2) in a living body. In the latter half of the nineteen eighties, an ultrafine fiber with monofilament size of 0.8 dtex or less (Patent document 3) was introduced into medical field (Patent document 4). Medical implements having good pliability and easy handling which were made of ultrafine polyester fibers were developed (Patent document 5-9).

Methods of manufacturing ultrafine fibers will be illustrated briefly. Generally, there are limitations to make a fiber fine by spinning or drawing, when polyester polymer is used, or even when other kinds of polymers are used. Namely, it is difficult to manufacture fine fibers having monofilament size of 0.8 dtex or less by 'direct spinning method'. Therefore, fibers having monofilament size of 0.8 dtex or less are called ultrafine fiber. In order to overcome the difficulty, a method was developed by Mr. Okamoto (Patent document 3), in which the procedure is to fix bundled fibers by a soluble polymer (thus the bundle is made up to one fiber), and to draw it close to the limit, then to remove the soluble polymer so as to obtain multi filaments. Mr. Okamoto's method was named 'sea-island method'. According to Mr. Okamoto's method, bundled fibers are fixed by polystyrene. After Okamoto's method, other methods such as 'dividing type method' were proposed. One of which is as below; polyamide polymer and polyester polymer are extruded as one fiber, and then, separated into two kinds of fine fibers. Polyamide, polyethylene, polylactic acid, super high molecular weight polyalkylene condensation polymer, polyethyleneglycol polyethylene terephthalate copolymer, polyethyleneglycol compounds or 5-sodium sulfonate isophthalate polyethylene terephthalate copolymer were also used for this purpose. These polymers above mentioned can be removed by dissolving by alkaline aqueous solution. Nylon 6 can be removed by dissolving by such organic solvent as trichloroethylene or toluene.

Ultrafine multi filaments manufactured by 'sea-island type method' or 'dividing type method' are widely applied for clothing or furniture, and are favorably accepted for their suede-like touch.

Medical materials made of multi filaments have many advantages. For example, in case of conventional artificial vascular grafts made of polyester fibers of ordinary thickness, when a graft fabric is woven so tightly as to prevent blood leakage, the woven fabric becomes rigid like as tent fabric. On the contrary, by using multi filaments, graft fabric remains flexible. Therefore, in clinic, handling of a graft made of multi filaments is easy, and moreover, that makes it possible to create a condition which water can leak, but blood cannot leak.

Automobile cells have a tendency to gather along ultrafine fibers. This phenomenon is biologically understood as 'thigmotaxis'. The inventor of the present invention found that when ultrafine fibers are used for medical materials in vivo, fibroblasts gather actively along the fibers, and act mainly for tissue healing. Therefore, wound healing promotion can be expected by use of ultrafine fibers.

However, in case of such medical materials as artificial vascular grafts, suture threads, artificial pericardiums, or tissue covering materials, the inventor of the present invention found that when multi ultrafine fibers were used, foreign body reaction occurs more intensely after in vivo implantation for a long period of time longer than 2 months, while foreign body reaction is negligible if conventional polyester fibers of ordinary size were used. When foreign body reaction is stronger, 'thigmotaxis' is less, that is, healing acceleration cannot be expected. This inconvenience has not become public known yet.

There is another inconvenience. When medical materials made of ultrafine fibers are implanted in vivo longer than 2 months, calcification is seen around multi filaments. The inventor of this invention is the first that found these phenomena i.e. foreign body reaction and calcification, although they are not public known yet. Therefore, there is no information reported about the countermeasures to reduce the phenomena.

Furthermore, when a medical material made of ultrafine fibers such as an artificial vascular graft is implanted into a site where high tension is loaded by pulse, ultrafine fibers come to arrange tightly, and interstices among the fibers become narrower. Namely, a closest packing state can be easily formed. In said state, cells such as fibroblasts, which are expected to promote tissue healing, cannot enter the interstices among ultrafine fibers. Consequently, healing delay is caused.

In comparison with fibers of ordinary thickness, ultrafine fibers provide a very good scaffold to fibroblasts, since surface area of ultrafine fibers is remarkably wider than that of ordinary fibers. As a result, infiltration and proliferation of fibroblasts are accelerated, and desirable healing tissue is quickly formed on the site where it is needed by power of vivo itself. If ultrafine fibers are in a closest packing state, characteristic of 'thigmotaxis' of cells cannot be induced, and good healing promotion cannot be obtained. Regarding said inconvenience mentioned above is not public known yet.

As mentioned above, application of ultrafine fibers developed for clothing to medical use is not preferable. Especially, medical materials implanted in vivo for long term sometimes cause inconvenience mentioned above, since these problems are not solved yet.

Accordingly, causes of said inconvenience, i.e. foreign body reaction and calcification are to be investigated urgently. Regarding afore mentioned ultrafine fibers manufactured by 'sea-island type method' or 'dividing type method', it is necessary to remove excessive polymer which remains at 'sea region' after spinning. To remove the remaining polymer, solvent which can easily dissolve these polymer is used. (Polyester polymer does not need this procedure, since polyester is harmless in a living body.) In case of ultrafine fibers, however, earnest investigation by the inventor of the present invention revealed that a very small amount of polymer is still left in 'sea region', even if any kinds of solvents are used.

For example, in case of use of copolymerized polyester of 5-sodiumsulfonate iesophthalate, which has come to be used recently as a compound for 'sea region', it can be removed easily by alkaline solvent. When fibers of ordinary thickness made of copolymerized polyester of 5-sodiumsulfonate iesophthalate were used, there is no influence by the remnant after dissolving the polymer. On the contrary, when ultrafine fibers made of copolymerized polyester of 5-sodiumsulfonate iesophthalate were used, the surface area of ultrafine fibers is much larger, and consequently, amount of the remnant is extremely more. This tendency becomes remarkable, in proportion to fineness of fibers. In other words, it is difficult to remove perfectly copolymerized polyester of 5-sodiumsulfonate iesophthalate from the ultrafine fiber surface, in molecular level.

When a medical material made of ultrafine fibers is implanted in vivo, cationic components, especially calcium cationic ions are attracted by sulfone group (-SO₃⁻) in copolymerized polyester of 5-sodiumsulfonate iesophthalate, and the calcium compounds accumulate on the fiber surface. The inventor of the present invention clarified that this phenomenon is a cause of calcification which is observed at interstices of ultrafine fibers. Light microscopic observation revealed that calcification had initiated from the core part of the bundle of ultrafine fibers. This finding indicates the difficulty of perfect washing to remove excessive polymer, especially from the core part of ultrafine fiber bundle.

Even when polystyrene, which has less cytotoxicity and no ionic problem, was used as 'sea' polymer, foreign body reaction and calcification are observed. Ernest investigation about the causes of above mentioned problems by the inventor of the present invention revealed that such solvents as trichloroethylene or toluene has infiltrated into ultrafine fiber surface, and that a small amount of the solvent causes foreign body reaction.

When alkaline solvent or acidic solvent such as formic acid is used, ester bond of polyester is hydrolyzed, and -COOH group is exposed on the fiber surface. Depending on conditions, -COOH group can be changed to -COO-, which can be a cause of calcification as well as -SO₃⁻ existing in sulfone group of copolymerized polyester of 5-sodiumsulfonate iesophthalate.

Further, alkaline solvent or acidic solvent are also absorbed on polyester fiber surface, and can be a cause which induces foreign body reaction. Such phenomenon is negligible when fibers of ordinary thickness were used, however, the inventor of the present invention clarified that when ultrafine fibers were used, said phenomenon exceeds a negligible level, because of marked increase of surface area of ultrafine fibers.

Further, in the present invention, problems which occur during the preparation process of raw materials for medical use are clarified. In general, a neutral detergent is used in order to wash the raw material. To cope with this problem, careful attention is needed. The reason can be explained as follows. While ultrafine fibers are being washed during preparation process, the detergent is absorbed from the surface of ultrafine fibers, due to extremely wide ultrafine fiber surface, as afore mentioned. And the absorbed detergent displays cytotoxicity. This point is also clarified in the present invention. In general, washing of raw material is done as the final course of preparation process, and this phenomenon occurs seriously only by using medial materials made of ultrafine fibers, although cytotoxicity is not recognized in case of fibers of ordinary thickness. Therefore, it is necessary to select a suitable and non -absorbable detergent. This finding is not pointed out up to the present time.

Accordingly, it is clear that indispensable conditions to use ultrafine fibers as a medical material for long-term in vivo implantation are, firstly, the use of polyester fibers to which any other synthetic polymer material than polyester are not adhered or absorbed, and secondarily, the use of a non - absorbable solvent, whether it is organic, alkaline or acidic.

Ultrafine fibers manufactured by 'sea-island type method' or 'dividing type method' are excellent for clothing or furniture, but application of ultrafine fibers manufactured by these methods to medical materials for long-term in vivo implantation is difficult to satisfy such conditions mentioned above, because ultrafine fibers are exposed to said solvents during manufacturing process. On the contrary, when ultrafine fibers are manufactured by 'direct spinning method', there is no risk of being exposed to these solvents, that is, ultrafine fibers can satisfy the conditions above mentioned.

Of course, ordinary fibers of more than 8 micron in thickness are generally manufactured by 'direct spinning method', and these solvents are not needed. Therefore, ordinary fibers of more than 8 micron satisfy the conditions above.

In the meanwhile, fiber manufacturing technique other than 'sea-island type method' or 'dividing type method' has improved, for example, a method of irradiation of an infrared ray or carbonic acid laser beam around a spinneret, so as to heat fibers at the point immediately after extrusion of fibers, which is called 'direct spinning method', was developed. Thus monofilaments having 0.8 dtex or less can be obtained directly. However, in case of manufactured monofilaments by said 'direct spinning method', it is necessary to set up spinning conditions minutely. Therefore, compared with 'sea-island type method' or 'dividing type method', mass production by this method is difficult. Further, another problem of weakness of the fibers is pointed out. Therefore, use of ultrafine fibers manufactured by 'direct spinning method' was limited to ex vivo use such as filters, and not used for medical materials for long-term in vivo implantation.

When ultrafine fibers are manufactured by 'direct spinning method', satisfactory strength of the product is required for long-term in vivo implantation as medical materials. Generally, it is known that strength of ultrafine fibers manufactured by 'direct spinning method' is less than that of ultrafine fibers manufactured by 'sea-island type method' or 'dividing type method'.

Accordingly, the inventor of the present invention points out that it will be necessary to use polyester ultrafine fiber which has 0.8 dtex or less and 4.0cN/dtex tensile strength or more, for medical implants manufactured by 'direct spinning method'. Said tensile strength value is a level obtained by ordinary spinning method.

In present invention, various kinds of tests for long-tem in vivo implantation had been carried out on ultrafine fibers for medical implants manufactured by 'direct spinning method', and following discoveries were made.

In general, in manufacturing process of polyester fiber, antimony is used as a catalyst. For clothing, polyester fiber is manufactured by said method. Since 1957, polyester fiber has been used worldwide for medical application, and an antimony catalyst has been used. In case of polyester of ordinal thickness, foreign body reaction sometimes occurs, but the gravity was negligible. The inventor of the present invention found that when ultrafine fibers were used, foreign body giant cells have grown around the fibers. Even when ultrafine fibers manufactured by 'direct spinning method' were used, foreign body reaction occurred in spite of no washing by alkaline, acidic solution, or a neutral detergent. The inventor of the present invention investigated a cause of the phenomenon, and points out that since ultrafine fiber has extremely wide surface area, some toxic matter which exists inside the fiber comes to spread onto the fiber surface, and exceeds an activating level of giant cells. In other words, the cause of said problem is considered to be an unidentifiable matter which the fiber contains. This inconvenience is not public known yet, however, it must be an unavoidable problem for medical materials for long-term in vivo implantation..

Investigation about toxic matter existing inside of the fiber had been carried out, and it revealed that antimony which was used as a catalyst was causal. In case of polyester fiber for clothing, a small amount of antimony catalyst remains inside, but foreign body reaction is negligible, while in case of ultrafine fibers, which have wide surface area, antimony spread widely on fiber surface.

The inventor of the present invention has investigated a cause of the phenomenon, and points out that ultrafine fibers for medical materials must be manufactured by a catalyst with less toxicity than antimony. In the present invention, since germanium or titanium has less toxicity than antimony, ultrafine polyester fibers were manufactured by a catalyst of germanium, and tests for implantation in vivo were carried out. The results showed that there is almost no foreign body reaction.

### PRIOR ARTS

### Non patent document

Non Patent Document 1: Greech AB Jr. et al: Vascular prostheses, report of the committee for the study of vascular prostheses of the society for vascular surgery. Surg 41(1); 62-80, 1957.
Non Patent Document 2: Noishiki Y.: Biochemical response to dacron vascular prosthesis. J Biomed Mat Res., 10(5) 759-67, 1976
Non Patent Document 3: Noishiki Y. et al; Evaluation of anew vascular graft prosthesis fabrication from ultrafine fiber, XXXII Trans Am Soc Artif Organs, 309-314, 1986

### Patent Documents

Patent Document 1: U.S.P 3,865,678 publication
Patent Document 2: JP S61-4546B2 publication
Patent Document 3: JP H5-48132B2 publication
Patent Document 4: JP H4-59901B2 publication
Patent Document 5: JP H4-59899B2 publication
Patent Document 6: U.S.P 4,695,280 publication
Patent Document 7: EP 128,741 publication
Patent Document 8: U.S.P 5,250,245 publication
Patent Document 9: JP 2005-325494A publication
Patent Document 10: JP 2007-100253A publication
Patent Document 11: PCT/JP2012/051567

### BRIEF SUMMARY OF THE INVENTION

### OBJECT OF THE INVENTION

As mentioned above, the problems to solve by the inventor is to provide medical materials for long-term in vivo implantation which are made of polyethylene terephthalate ultrafine fibers without causing foreign body reaction or calcification, displaying "thigmotaxis" that cells gather along ultrafine fibers, and by having good pliability and easy handling which a conventional ultrafine fiber also has.

### BRIEF ILLUSTRATION OF THE INVENTION

The inventor of the present invention earnestly investigated to solve above mentioned problems and repeated experiments, and found that the problems can be solved by settling following conditions, that is, firstly the use of polyester fibers. Secondly, the use of not absorbable solvents, whether they are organic, alkaline or acidic. Thirdly, selection of a catalyst which does not have cytotoxicity during the manufacturing of ultrafine fibers. In the fourth place, single fiber thickness of ultrafine fiber is 0.8 dtex or less, having 4.0cN/dtex or more tensile strength. In the fifth place, these ultrafine fibers do not cause a closest packing condition. And the inventor of the present invention accomplished the present invention.

That is, the present invention can be illustrated as follows.
(1) A medical material for in vivo implantation comprising a polyethylene terephthalate ultrafine fiber having single fiber thickness of 0.8 dtex or less which is manufactured using a polyethylene terephthalate polymer chip by 'direct spinning method', wherein the polyethylene terephthalate polymer chip is manufactured using germanium or titanium as a catalyst.
(2) The medical material for in vivo implantation of (1), wherein the ultrafine fiber has tensile strength of 4.0cN/dtex or more.
(3) The medical material for in vivo implantation of (1) or (2), wherein the bundle of ultrafine fibers is treated by false twisting process, and the bundle has one or more micro-crimps per mm on average.
(4) The medical material for in vivo implantation of anyone of (1) to (3), wherein the medical material for in vivo plantation is at least one selected from the group consisting of artificial vascular grafts, patch material, artificial heart valves, stent grafts, artificial pericardium, suture threads, tissue covering materials, tissue reinforcing materials, tissue coating materials, tissue recovering base materials, tissue repairing materials, anti-adhesive materials, clips, fabrics or hemostatic materials.

### EFFECT OF THE INVENTION

The medical materials for long-term in vivo implantation of the present invention are made of polyethylene terephthalate ultrafine fibers, which do not cause foreign body reaction or calcification. They show a special characteristic of "thigmotaxis" which is mother nature of automobile cells, i.e., a tendency to gather along ultrafine fibers. Due to this tendency, ultrafine fibers provide a suitable scaffold for infiltrating cells into the interstices among these fibers, resulting in desirable infiltration and proliferation of fibroblasts which play an important role for tissue healing. Thus, infiltrated cells can create new organs in corporation with ultrafine fibers. Said medical materials made of these ultrafine fibers can be accepted as part of a living body. The materials show good pliability and easy handling, which conventional ultrafine fibers also have.

### PREFERRED ENBODIMENT OF THE INVENTION

The present invention will be illustrated in detail as follows.
As the medical material for long-term in vivo implantation of the present invention is at least one medical material for in vivo implantation selected from the group consisting of artificial vascular grafts, patch material, artificial heart valves, stent grafts, artificial pericardium, suture threads, tissue covering materials, tissue reinforcing materials, tissue coating materials, tissue recovering base materials, tissue repairing materials, anti-adhesive materials, clips, fabrics or hemostatic materials. In general, these medical materials are seldom removed from a patient's body after implantation, and left in vivo for long-term at the implanted site.

As previously mentioned, regarding thickness of fiber, a fiber whose single fiber thickness is 0.8 dtex or less is called as ultrafine fiber. In the present invention, ultrafine fiber whose single fiber thickness is 0.8 dtex or less is used, but desirable single fiber thickness is 0.5 dtex or less, and more desirable single fiber thickness is 0.3 dtex or less. Regarding the limit of thickness, since theoretically possible thickness by current technique is 0.01 dtex, the lowest limit of thickness can be set up to 0.01 dtex.

As a catalyst of polyester polymer, germanium or titanium has less toxicity than antimony. The present invention, germanium or titanium catalyst is used.

Such medical materials for in vivo implantation as artificial vascular graft are sometimes implanted in the site where high tension is loaded by pulse. In said case, when ultrafine fibers are used, the same strength as fibers of ordinary thickness is also required. Generally, the strength of ultrafine fibers manufactured by 'direct spinning method' are weaker compared with ordinal fibers. In the present invention, ultrafine fibers are required to have tensile strength higher than 4.0 cN/dtex.

When medical materials are used in a place where high tension is loaded, there is one more anxiety. That is, if bundled ultrafine fibers are arranged in a closest packing state, the interstices among the ultrafine fibers become too narrow, therefore, cells can hardly infiltrate into ultrafine fibers. To prevent this phenomenon, ultrafine fibers are required to be in a dispersed condition even they are at a place with high tensile stress.

In the present invention, to prevent such phenomenon, elaborative false twisting of ultrafine fibers was introduced. Basically, how to make false twisting is not restricted. A method disclosed in PCT/JP2012/051567 (Patent Document 12) will be applicable. The inventor of this invention improved this method by providing micro crimps evenly to ultrafine fibers without any damage.

The present invention will be illustrated definitely by Examples, however, not intending to restrict the present invention to these Examples.

### EXAMPLES

As medical materials for long-term in vivo implantation, artificial vascular grafts, patch materials, artificial heart valves, stent grafts, artificial pericardium, suture threads, tissue covering materials, tissue reinforcing materials, tissue coating materials, tissue recovering base materials, tissue repairing materials, anti-adhesive materials, clips, fabrics or hemostatic materials can be mentioned. An artificial vascular graft is selected as a representative of these medical materials, and implantation of it into the thoracic descending aorta of a dog is carried out.

### Example 1

Both germanium and titanium are characterized as less toxicity, and in Examples. Polyethylene terephthalate polymer manufactured using a germanium catalyst was adopted. The ultrafine fibers which the inventor used were manufactured by 'direct spinning method', heating around a spinneret by an infrared ray irradiation. Thickness of the manufactured ultrafine fiber is 0.3 dtex, which is a representative example of less than 0.8 dtex. Regarding strength of fiber, a fiber of 4.2cN/dtex, which is manufactured intending to be higher than 4.0cN/dtex, is used. False twisting is performed on the manufactured ultrafine fiber in a bundled state. As the method for false twisting, the method disclosed in Patent Document 12 is used.

As one example of a medical material for long-term in vivo implantation, experiments were carried out by using an artificial vascular graft. Ultrafine fibers of thickness of 0.3dtex were used as the woofs and another fiber having ordinary thickness of 1.2 dtex was used for the warps. The graft was manufactured using a germanium catalyst and direct spinning method. By combination of these fibers, a woven-type artificial vascular graft of 8mm inner diameter, whose water permeability by ANSI/AAMI standard is 150 ml, was prepared.

Said prepared artificial vascular graft was cut to specimens of 5 cm in length and implanted into the thoracic descending aorta of a dog. Six months after implantation, the specimens are removed and fixed with 10% formalin. Then sections for light microscope observation were prepared. The sections are dyed by hematoxylin and eosin and observed by a microscope by 100-400 magnifications. Consequently, numerous fibroblasts had infiltrated into the interstices of ultrafine fibers, and a new wall of a blood vessel had formed with the ultrafine fibers as a frame. And around ultrafine fibers, no foreign body giant cells were observed. Further, no calcification was observed, either.

### Example 2

Using a germanium catalyst, the same polyethylene terephthalate polymer, as to Example 1 was used, and 2 kinds of ultrafine fibers were adopted. The thickness of the two kinds of fibers was approximately 0.15 dtex and 0.1 dtex.

These fibers were used as the woof, and fibers having ordinary thickness of 1.2 dtex used in Example 1 was used for the warp. Thus 2 kinds of woven type artificial vascular graft were prepared.

By the same method as Example 1, the prepared artificial vascular grafts were implanted into the thoracic descending aorta of a dog. Six months after implantation, the specimens were removed and observed by a light microscope. There was no foreign body reaction such as giant cell infiltration. No calcification was observed around said two artificial vascular grafts.

### Example 3

Burst strength of the artificial vascular graft of Example 1 was measured in accordance with the ANSI/AAMI standard, which resulted in 18.5 kg. Burst strength of a commercially available artificial vascular graft is more than 10.0 kg. Therefore, it is obvious that the strength of an artificial vascular graft in which the ultrafine fibers as the woof is sufficient to endure as medical materials for in vivo implantation. There was no sign of ultrafine fiber deterioration.

### Comparative Example 1

Polyethylene terephthalate polymer fiber of 1.2 dtex, which is ordinary in thickness, was made using a germanium catalyst according to a conventional manufacturing method. Said fiber is used as both for the warp and the woof, and a woven type artificial vascular graft of 8 mm inner diameter was prepared. Water permeability of the artificial vascular graft is 150 ml.

Said prepared artificial vascular graft was cut to 5 cm length specimens, and implanted into the thoracic descending aorta of a dog in accordance with the method of Example 1. Six months after implantation, the specimens were removed and fixed by 10% formalin. Thus sections for light microscope observation were prepared. The sections were dyed by hematoxylin and eosin, and observed under a light microscope by 100-400 magnifications. The interstices among ultrafine fibers had adequate width, and numerous fibroblasts infiltrated into these interstices of ultrafine fibers. A new wall of a blood vessel was formed with the ultrafine fibers as a frame. Although foreign body reaction was not remarkable around ultrafine fibers, one foreign body giant cell in 20 views was observed, which was in ignorable level. And no calcification can be observed, either.

### Comparative Example 2

Commercially available fibers of ordinal thickness of 1.2 dtex were adopted, which were made of polyethylene terephthalate polymer using an antimony catalyst according to conventional manufacturing method. Further, an ultrafine fiber was manufactured by 'sea-island method' using the same polyethylene terephthalate polymer. As the 'sea' part, copolymerized polyethylene terephthalate of 5-sodiumsulfonate isophthalate was used. The thickness of a monofilament, after the sea part polymer was removed, was 0.3 dtex.

The fibers of 1.2 dtex were used as the warp and the fiber of 0.15 dtex was used as the woof, and an artificial vascular graft of 8mm inner diameter was prepared. Water permeability of the artificial vascular graft was 150 ml. The vascular graft was washed by shaking with pressure in order to remove the remaining polymer in the sea part using 0.25% NaOH aqueous solution, and then washed by distilled water after neutralization. The prepared artificial vascular graft was cut to 5 cm in length, and implanted into the thoracic descending aorta of a dog in accordance with the method of Example 1. Six months after implantation, the specimens were removed, and fixed by 10% formalin. Then sections for light microscopic observation were prepared. The sections were dyed by hematoxylin and eosin, and observed by a light microscope by 100-400 magnifications. As a result, foreign body giant cells were observed surrounding the ultrafine fibers. By observation of 100 magnification, 6 foreign body giant cells on average were observed in 1 view. This result showed that foreign body reaction against these fibers had occurred. The same segment was dyed in accordance with Von Kossa method. And the interstices among ultrafine fibers were dyed black colour, which indicates calcium deposition.

### Comparative Example 3

The artificial vascular graft prepared in Comparative Example 2 was used. Elemental analysis by NMR was carried out on the specimen of the artificial vascular graft before implantation. At five points which were chosen from the specimen by random sampling, 50-100 ppm of sulfur was detected. According to the analytical result, it is indicated that sulfur component in sulfone group of the copolymerized polyethylene terephthalate of 5-sodiumsulfonateisophthalated remained in the interstices of the fibers. That is, even if washing by alkaline solution was carried out, sea-part was not cleaned perfectly. It can be considered that calcium cationic ions are attracted because sulfone group remains.

### Comparative Example 4

The specimen of the artificial vascular graft before implantation, which was used in Comparative Example 2, was washed by alkaline solution repeatedly, then elemental analysis by NMR analytical method is carried out again. Actually the same washing was repeated 3 times and 5 pieces were picked up by random sampling. Since 5-20 ppm of sulfur was detected from all pieces, it is clear that repetition of washing by alkaline solution cannot remove 'sea-part' polymer perfectly.

### Comparative Example 5

Ultrafine fibers were manufactured using the same polymer as Example 1. Thickness of the ultrafine fibers was 0.3 dtex. Strength of the fibers was 3.0cN/dtex, wherein spinning was carried out without considering fiber strength. An artificial vascular graft of 8mm inside diameter was prepared using these ultrafine fibers as the woof, and fibers with ordinary thickness of 1.2 dtex as the warp. Burst strength of the prepared artificial vascular graft was measured in accordance with burst test method of ANSI/AAMI standard, and result of 9.5 kg was obtained. Burst strength of artificial vascular grafts which are commercially available was more than 10.0 kg. It was obvious that strength of the prepared artificial vascular graft which was made of the ultrafine fibers manufactured with fiber strength out of consideration was not enough for medical materials for long-term implantation.

### Example 4

The same as Example 1, using the same the woof and the warp as Example 1, an artificial vascular graft for in vivo implantation of 8mm inner diameter was prepared. In order to keep ultrafine fibers separate each other, an ordinary false twisting process according to a method disclosed in PCT/JP2012/051567 (Patent Document 12) was performed on ultrafine fibers used as the woof. Water permeability of the artificial vascular graft prepared as above was 150 ml.

The same as Example 1, the prepared artificial vascular graft was implanted into the thoracic descending aorta of a dog. Six months after implantation, the specimens were removed, and prepared for light microscopic observation. No foreign body reaction i.e., no giant cell gathering, was observed around ultrafine fibers used as the woof nor ordinary fibers in thickness used as the warp. However, ultrafine fibers used as the woof were tightly contacted with each other, and the sectional view of these ultrafine fibers indicated a closest packing state of ultrafine fibers. Fibroblast infiltration was seldom observed in the interstices among these ultrafine fibers. From the result, it is suggested that the interstices among the ultrafine fibers should be maintained wide enough for fibroblasts infiltration to induce natural healing. A woven artificial vascular graft was prepared by a conventional method. When the graft was implanted in a site where high tension was loaded by pulse, ultrafine fibers came to arrange tightly, and interstices among the fibers became narrower, which showed a closest packing state. This phenomenon was not so serious in ordinary fibers in thickness as the warp. In case of ultrafine fibers as the woof, this phenomenon was remarkable. Accordingly, when the ultrafine fibers are used at a place where tensile stress is loaded in vivo, it is desirable to have each fiber arranged into a separable state by future improvement of false twisting.

### Example 5

In this Example, a suitable degree of false twisting to prevent a closest packing state of ultrafine fibers was investigated. The ultrafine fiber used as the woof in Comparative Example 5 was adopted. Various degrees of false twisting process were tested, changing twisting frequency and twisting speed, in order to provide micro-crimps effectively to the ultrafine fiber. Adequate distribution of micro-crimps was obtained at more than 4000/m twisting in a bundled form of ultrafine fibers. The bundle had one or more micro-crimps per mm on average. As a result, there were wide and suitable interstices among ultrafine fibers, even if such medical materials as artificial vascular grafts were placed under a condition of 120 mmHg of inner pressure. The twisted bundle could prevent a closest packing state of the ultrafine fibers.

### POSSIBILITY FOR THE INDUSTRIAL USE

As mentioned above, medical materials for long-term in vivo implantation were developed by using polyethylene terephthalate ultrafine fibers of 0.8dtex or less in thickness, which were manufactured by direct spinning and drawing of the polymer using a germanium or titanium catalyst. Medical materials made of said fibers can prevent foreign body reaction and calcification after implantation. Therefore, the utility value of this invention is high enough.

## Claims

1. A medical material for in vivo implantation comprising a polyethylene terephthalate ultrafine fiber having single fiber thickness of 0.8 dtex or less which is manufactured using a polyethylene terephthalate polymer chip by direct spinning method, wherein said polyethylene terephthalate polymer chip is manufactured using germanium or titanium as a catalyst.

2. The medical material for in vivo implantation of claim 1, wherein the ultrafine fiber has tensile strength of 4.0cN/dtex or more.

3. The medical material for in vivo implantation of claim 1 or claim 2, wherein the bundle of ultrafine fibers is treated by false twisting process and the bundle has one or more micro-crimps per mm on average.

4. The medical material for in vivo implantation of anyone of clams 1 to 3, wherein the medical material for in vivo plantation is at least one of those selected from the group consisting of artificial vascular grafts, patch material, artificial heart valves, stent grafts, artificial pericardium, suture threads, tissue covering materials, tissue reinforcing materials, tissue coating materials, tissue recovering base materials, tissue repairing materials, anti-adhesive materials, clips, fabrics or hemostatic materials.
